# EUROPEAN PATENT SPECIFICATION

(11) **EP 1 559 754 B1**
(45) Date of publication and mention of the grant of the patent: **06.12.2006**
(21) Application number: 03769950.1
(22) Date of filing: 29.10.2003
(51) Int. Cl.: C09D 11/00, B41M 5/00, B41J 2/01, C07C 49/92, C07D 333/16, C07F 5/00, C09K 11/06

(54) **INK COMPOSITION**
TINTENZUSAMMENSETZUNG
COMPOSITION D'ENCRE

(30) Priority: 06.11.2002 JP 2002322126
(43) Date of publication of application: 03.08.2005
(73) Proprietor: NIPPON KAYAKU KABUSHIKI KAISHA, Tokyo 102-8172 (JP)
(72) Inventor: KITAYAMA, Yasuyuki, Hartsdale, NY 10530 (US); KATOH, Yosinori, Saitama-shi, Saitama 331-0823 (JP); KIYOYANAGI, Noriko, Kita-ku, Tokyo 115-0052 (JP)
(74) Representative: Wablat, Wolfgang
(86) International application number: PCT/JP2003/013839
(87) International publication number: WO 2004/041945

(56) References cited:
- JP-A- 1 026 583
- JP-A- 8 239 609
- JP-A- 10 017 571
- JP-A- 2002 348 508
- JP-A- 2002 356 632
- JP-A- 2003 261 809
- PATENT ABSTRACTS OF JAPAN vol. 1997, no. 01, 31 January 1997 (1997-01-31) & JP 08 239609 A (TOYO INK MFG CO LTD), 17 September 1996 (1996-09-17)
- PATENT ABSTRACTS OF JAPAN vol. 013, no. 208 (C-596), 16 May 1989 (1989-05-16) & JP 01 026583 A (MITSUI TOATSU CHEM INC), 27 January 1989 (1989-01-27)
- PATENT ABSTRACTS OF JAPAN vol. 1998, no. 05, 30 April 1998 (1998-04-30) & JP 10 017571 A (TOYO INK MFG CO LTD), 20 January 1998 (1998-01-20)

## Description

### Technical Field

The present invention relates to a recording material. The present invention relates, particularly, to an ink composition which provides a print record which is colorless and cannot be therefore invisible to the naked eye under visible light, but develops a color and is visible under the radiation of ultraviolet rays.

### Background Art

A tris(thenoyltrifluoroacetonate)europium complex, a tris(benzoyltrifluoroacetonate)europium complex and the like are known compounds which are colorless under visible light but emit red light under the radiation of ultraviolet rays and are applied to various inks (see, for example, Patent Documents 1 and 2). However, among these compounds, the complexes superior in stability and durability have insufficient solubility in solvents, especially, water-containing solvents and are therefore limited in its applications.

As to applications to solvents containing water, examples using various europium complexes are described (see, for example, Patent Document 3). However, sufficient solubility in a water-containing solvent has not been obtained so far. Particularly if the content of water in an ink medium is increased, the problems arises that, for example, the precipitation of the ink is caused by long-term storage and the intensity of emitted light is decreased. Also, there is the problem that a coating film after printing is sticky and printedmatter(e.g.,charactersandbarcodes)isrubbed,bringing about difficult reading.

In the meantime, complexes of rare earth elements coordinated with quaternary β-diketones have been already known as ultraviolet absorbers. (See, for example, Patent Document 4)

Patent Document 1: Japanese Patent Application Laid-Open (JP-A) No. 2000-160083 (pages 2 to 4)
. Patent Document 2: JP-A No. 8-253715 (pages 3 to 5)
. Patent Document 3: JP-A No. 2002-37791 (pages 3 to 5)
. Patent Document 4: USP No. 3254103

### Problems to be solved by the Invention

It is an object of the present invention to provide an ink composition which has good ink stability, uses water or a mixed solvent including water that is safe to human bodies and environments as a medium and emits red light when irradiated with ultraviolet rays.

### Disclosure of the Invention

The inventors of the present invention have made earnest studies to solve the above problem and as a result, found that a specific europium compound (complex) solves the above problem, to complete the present invention.

Accordingly, the present invention relates to:
(1) an aqueous ink composition comprising a europium compound (A) represented by the following formula (1), a binder (B) and an aqueous medium (C): 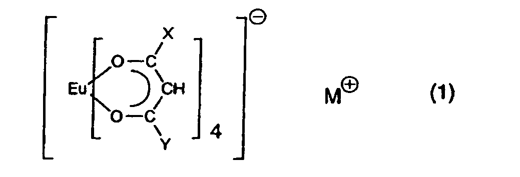 wherein X represents a benzene cyclic group, a naphthalene cyclic group, a pyridine cyclic group, or a thiophene cyclic group which may have a substituent, Y represents a fluorinated hydrocarbon group having 1 to 10 carbon atoms and M represents an alkali metal or an alkali earth metal;
(2) an aqueous ink composition according to the above (1), wherein X is a benzene cyclic group or a thiophene cyclic group ;
(3) an aqueous ink composition according to the above (1) or (2), wherein Y is a trifluoromethyl group;
(4) an aqueous ink composition according to any one of the above (1) to (3), wherein M is an alkali metal;
(5) an aqueous ink composition according to any one of the above (1) to (4), wherein (B) is a water-soluble high-molecular compound;
(6) an aqueous ink composition according to any one of the above (5), wherein (B) ispolyvinylalcohol, modifiedpolyvinylalcohol or a polymer having a cyclic amide group in a molecular structure;
(7) an aqueous ink composition according to any one of the above (1) to (6), wherein the content of (A) is 0.1 to 4% by weight, the content of (B) is 0.1 to 15% by weight and the content of (C) is 73.5 to 99.8% by weight; and
(8) a colored body treated with the aqueous ink composition according to any one of the above (1) to (7).

### Best Mode for Carrying Out the Invention

The present invention will be explained in detail. The europium compound (A) in the present invention is one represented by the aforementioned formula (1). X in the formula (1) represents a benzene cyclic group, naphthalene cyclic group, a pyridine cyclic group or a thiophene cyclic groups. Preferable examples include a benzene ring, naphthalene ring, pyridine ring and a thiophene ring. Examples of the substituent which X may have include an alkyl group, alkoxyl group, aromatic cyclic group, heterocyclic group, amino group, alkylamino group, dialkylamino group, hydroxyl group, aralkyl group and halogen atom. Preferable examples include a halogen atom and alkyl group.

Y represents a fluoride hydrocarbon group having 1 to 10 carbon atoms. Specific examples of the fluoride hydrocarbon group include perfluoroalkyl groups such as a trifluoromethyl group, pentafluoroethyl group, heptafluoropropyl group and heptadecafluorooctane; monofluoromethyl groups, difluoromethyl groups, trifluoroethyl groups, tetrafluoropropyl groups and octafluoropentyl groups. Preferable examples include perfluoroalkyl groups such as a trifluoromethyl group, pentafluoroethyl group and heptadecafluorooctane group. More preferable examples include a trifluoromethyl group.

M represents an alkali metal or an alkali earth metal. M is necessary to neutralize the plus charge of europium. The amount of M to be required per 1 mol of europium is 1 mol in the case of an alkali metal and 1/2 mol in the case of an alkali earth metal. Examples of the alkali metal include lithium, sodium, potassium, rubidium, cesium and francium. Examples of the alkali earth metal include beryllium, magnesium, calcium, strontium, barium and radium. Preferable examples include lithium, sodium, potassium, magnesium and calcium and more preferable examples include lithium, sodium and potassium.

In a particularly preferable combination of X, Y and M in the formula (1), X is a naphthalene cyclic group or a thiophene cyclic group, Y is a trifluoromethyl group and M is lithium or sodium.

The compound of the formula (1) may be synthesized in the following manner. For example, the compound is easily synthesized by adding, for example, europium perchlorate or europium chloride in an alcohol or acetone solvent in the presence of a diketone derivative and an alkali agent, such as sodium hydroxide or potassium hydroxide, selected as the M source and by running a reaction at usually 0°C to 80°C. The structure of the cation affords opportunity for formation of an unstable complex and a complex which cannot be isolated stably, but permits the presence of a complex which can be isolated stably under controlled pH and temperature.

Specific examples of the compound of the formula (1) are shown in Table 1. In Table 1, a benzene ring is represented by Ph, a naphthalene ring by Np, a pyridine ring by Py, a thiophene ring by Th, a furan ring by Fu and a benzyl group by Bz.

**Table 1**

| Compound No. | X | Y | M |
|---|---|---|---|
| 1 | 2-Th | CF3 | Li |
| 2 | 2-Th | CF3 | Na |
| 3 | 2-Th | CF3 | K |
| 4 | 2-Th | CF3 | Mg |
| 5 | 2-Th | CF3 | Ca |
| 6 | 2-Np | CF3 | Li |
| 7 | 2-Np | CF3 | Na |
| 8 | 2-Np | CF3 | K |
| 9 | 2-Np | CF3 | Mg |
| 10 | 2-Np | CF3 | Ca |
| 11 | Ph | CF3 | Li |
| 12 | Ph | CF3 | Na |
| 13 | Ph | CF3 | K |
| 18 | 3-Py | CF3 | Na |
| 19 | 2-Th | -CF2CF2CH3 | K |
| 20 | 2-Np | -CF2CF2CH3 | Na |
| 21 | Ph | -CF2CF2CH3 | K |
| 23 | 3-Py | F2CF2CH3 | Li |
| 24 | 2-Th | C8F17 | Na |
| 25 | 2-Np | C8F17 | K |
| 26 | Ph | C8F17 | Na |
| 28 | 3-Py | C8F17 | K |

The ink composition of the present invention contains at least one of the europium compounds represented by the formula (1) (or its mixture, the same as follows)

As the compound represented by the formula (1) or its mixture which is to be used in the preparation of the ink composition of the present invention, a compound having a small content of inorganic salts is preferable. When the compound or its mixture is utilized for an aqueous ink composition, a compound having a particularly small content of inorganic salts is preferable.

In order to obtain a dye which is more reduced in the content of inorganic salts as the ink composition of the present invention, there are a method using a reverse osmosis membrane, a method of washing with water or a method of cleansing treatment with water, optionally using a lower alcohol such as methanol, ethanol or isopropanol.

The content of the europium compound (A) of the formula (1) is 0.001 to 10% by weight and preferably 0.01 to 4% by weight in the ink composition of the present invention.

A preferable example of the binder (B) is an emulsion of a water-soluble high-molecular compound or a hydrophobic high-molecular compound.

Given as examples of the water-soluble high-molecular compound which may be used are water-soluble compounds including a polyethylene glycol, polypropylene glycol, polyethylene glycol/polypropylene glycol block copolymer, polyethyleneimine, methyl cellulose, methoxy cellulose, hydroxyethyl cellulose, carboxymethyl cellulose, sodium carboxymethyl cellulose, cellulose, polyvinyl alcohol (PVA), modified polyvinyl alcohols such as a carboxyl group modified polyvinyl alcohol and sulfonic acid group modified polyvinyl alcohol, polyvinyl pyrrolidone, polyvinyl pyrrolidone/vinyl acetate copolymer, polyacrylamide, polyacrylic acid, starch and its derivatives, casein, gelatin, water-soluble isoprene rubber, alkali salt of a styrene/maleic acid copolymer, methyl vinyl ether/maleic acid anhydride copolymer, iso (or diiso)butylene/maleic acid anhydride copolymer.

Examples of the emulsions of hydrophobic high-molecular compounds include emulsions of hydrophobic high-molecular compounds such as a styrene/butadiene (SB) copolymer, styrene/maleic acid anhydride copolymer, styrene carboxylate/butadiene (SB) copolymer, styrene/butadiene/acrylic acid copolymer, polyvinyl acetate, polyvinyl chloride, vinyl chloride/vinyl acetate copolymer, polystyrene, acrylic resin, acryl/styrene resin, polyacrylate, polyester, polycarbonate, polyurethane, polybutyral, epoxy resin, furan resin, vinyltoluene resin and rosin ester resin, and emulsions of these hydrophobic high-polymer compounds made to be self-emulsifiable by compounding a base or a polyethylene oxide in these compounds.

The binder (B) is preferably a compound which is itself solid at ambient temperature (about 5 to 35°C) and is preferably a water-soluble high-molecular compound. Also, the binder (B) is preferably a polyvinyl alcohol or a modified polyvinyl alcohol in consideration of the drying characteristics and stickiness of the coating film after printing. The binder (B) is more preferably a carboxyl group or sulfonic acid modified polyvinyl alcohol having a polymerization degree of 150 to 2000, a saponification value of 70 to 99 mol% and a carboxylic acid group or a sulfonic acid group in an amount of 0.01 to 5 mol% based on the copolymer.

Also, a polymer having a cyclic amide group in the molecular structure is suitable in consideration of the emission intensity of the coating film. Examples of the polymer having a cyclic amide group in the molecular structure include polymers obtained by homo-polymerizing monomers having a cyclic amide group such as N-vinyl pyrrolidone, N-vinyl piperidone and N-vinyl caprolactam or by copolymerizing these monomers with other monomers. Specific examples of these polymers include a polyvinylpyrrolidone, polyvinylcaprolactam, polyvinylpyrrolidone/acetic acid copolymer, vinylpyrrolidone/vinylcaprolactam copolymer, vinylpyrrolidone/vinylimidazole copolymer, vinylpyrrolidone/acrylic acid copolymer, vinylpyrrolidone/methacrylic acid copolymer and vinylpyrrolidone/3-methyl-1-vinylimidazolium salt copolymer. However, the binder is not limited to these polymers and any polymer may be used as the binder insofar as it has a cyclic amide group in its structure and is soluble in water or a mixed solvent of water and an organic solvent. A polymer is preferable in which the ratio of polymerization of the monomer having a cyclic amide group is 30% or more in the polymer. As the polymer having at least a cyclic amide group in the structure, those having a molecular weight of 4000 to 2000000, preferably 5000 to 1000000 and more preferably 5000 to 60000 may be used in consideration of the stability and viscosity of the ink composition.

The content of the binder (B) is 0.01% to 20% by weight and preferably 0.1% by weight to 15% by weight in the aqueous ink composition of the present invention in consideration of the stability of the ink and viscosity of the ink during use.

The aqueous medium (C) of the present invention is water or a mixture of water and a water-soluble organic solvent.

Specific examples of the water-soluble organic solvent which may be used include C1-C4 alkanols such as methanol, ethanol, 1-propanole, isopropanol, butanol, isobutanol, secondary butanol and tertiary butanol; lower carboxylic acids such as N,N-dimethylformamide or N,N-dimethylacetamide; (mono or di) lower alkylamides; lactams such as N-methylpyrrolidin-2-one and preferably four-membered to eight-membered lactams; cyclic ureas such as 1,3-dimethylimidazolidin-2-one or 1,3-dimethylhexahydro pyrimido-2-one and preferably five-membered or six-membered cyclic ureas; ketones and keto-alcohols in which the straight-chain has 4 to 7 carbon atoms such as acetone, methyl ethyl ketone and 2-methyl-2-hydroxypentan-4-one; ethers such as tetrahydrofuran and dioxane and preferably five or six-membered cyclic ethers; mono, oligo or polyalkylene glycols or thioglycols having a C2-C6 alkylene unit such as ethylene glycol, 1,2- or 1,3-propylene glycol, 1,2- or 1,4-butylene glycol, 1,6-hexylene glycol, diethylene glycol, triethylene glycol, dipropylene glycol, thiodiglycol, polyethylene glycol and polypropylene glycol; polyols (preferably triols with a carbon chain having 3 to 6 carbon atoms) such as glycerin and hexane-1,2,6-triol; C1-C4 alkyl ethers of polyhydric alcohols (preferably ethylene glycol or polyethylene glycol) such as ethylene glycol monomethyl ether, ethylene glycol monoethyl ether, diethylene glycol monomethyl ether, diethylene glycol monoethyl ether, triethylene glycol monomethyl ether and triethylene glycol monoethyl ether; and γ-butyrolactone. Among these compounds, methanol, ethanol, 1-propanol, isopropanol and N-methylpyrrolidin-2-one are preferable.

These water-soluble organic solvents, though the solvent used in the present invention is not limited to these solvents, may be used either alone or in combinations of two or more in consideration of the hygroscopicity and moisture retentivity of the solvent, the solubilities of materials to be compounded, penetrability and the viscosity of the ink.

The ink composition of the present invention has high stability and is free from any precipitation and a reduction in emission intensity with time even in a mixed solvent having a high water content. Accordingly, the ink composition of the present invention can be made into ink with reduced odor of the solvent and reduced flammability in consideration of an influence on environment. A water-soluble organic solvent may be used together to control the drying speed of the ink and the ratio of water in the mixed solvent (water/water-soluble organic solvent) is usually 10% by weight or more, preferably 10 to 90% by weight and more preferably 20 to 80% by weight.

A waterproofing agent may be added to the ink composition of the present invention in the application wherein waterproof properties is required. Examples of the waterproof agent which may be used include glyoxal, methylolmelamine and water-soluble epoxy compounds. Specific examples of the water-soluble epoxy compound include ethylenepolyethylene glycol diglycidyl ether, propylenepolypropylene glycol diglycidyl ether, glycerol polyglycidyl ether and lauryl alcohol glycidyl ether. The amount of the waterproofing agent is usually 0.5 to 50% by weight and preferably 1 to 30% by weight based on the amount of the binder.

Further, the ink composition of the present invention may contain an ink conditioner in an amount of 0 to 10% by weight and preferably 5% by weight or less. Examples of the ink conditioner include all components except for the aforementioned water, dye components and water-soluble organic solvent and specifically include an antiseptic, pH adjusting agent, antirust agent, water-soluble ultraviolet absorber, surfactant, dissolution adjuvant and specific resistance controller. The ink composition may also include chemicals for controlling surface tension, viscosity and conductivity. Examples of the antiseptic include sodium dehydroacetate, sodium sorbate, sodium 2-pyridinethiol-1-oxide, sodium benzoate and sodium pentachlorophenolate. As the pH conditioner, an optional material may be compounded insofar as it can control the pH of the ink in a range from 6 to 10 without adversely affecting the ink to be formulated. Examples of the pH conditioner include alkanol amines such as diethanolamine and triethanolamine, hydroxides of alkali metal elements such as lithium hydroxide, sodium hydroxide and potassium hydroxide, ammonium hydroxide and carbonates of alkali metals such as lithium carbonate, sodium carbonate and potassium carbonate. Examples of the antirust agent include acidic sulfites, sodium thiosulfate, ammon thioglycolate, diisopropylammonium nitrite, pentaerythritol tetranitrate and dicyclohexylammonium nitrate. Examples of the water-soluble ultraviolet absorber include sulfonated benzophenone and sulfonated benzotriazole. Examples of the surfactant include anionic surfactants, amphoteric surfactants, cationic surfactants and nonionic surfactants. Examples of the anionic surfactant which may be used include α-olefin sulfonate and polyoxyethylene alkyl ether acetate. Examples of the cationic surfactant include 2-vinylpyridine derivatives and poly 4-vinylpyridine derivatives. Examples of the amphoteric surfactant which may be used include lauryldimethylaminoacetic acid betaine, 2-alkyl-N-carboxymethyl-N-hydroxyethylimidazolinium betaine, coconut oil fatty acid amidopropyldimethylaminoacetic acid betaine. Examples of the nonionic surfactants which may be used include ether types such as polyoxyethylene nonylphenyl ether and polyoxyethylene octylphenyl ether, ester types such as polyoxyethylene oleic acid and polyoxyethylene oleate and acetylene glycol types such as 2,4,7,9-tetramethyl-5-decyne-4,7-diol and 3,6-dimethyl-4-octyne-3,6-diol. Examples of the dissolution adjuvant which may be used include urea and ε-caprolactam. Examples of specific resistance regulator include inorganic salts and organic salts. These ink conditioners may be used either alone or as a mixture.

The ink composition of the present invention may be prepared by mixing the europium compound (A) of the formula (1), the binder (B) and water, and as required, the water-soluble organic solvent and the ink conditioner, and by, as required, balancing the total amount with water finally. Also, if necessary, filtration may be carried out after the ink composition is obtained, to remove impurities. The pH of the ink is preferably about 6 to 10.

The ink composition of the present invention is most preferably used for ink jet recording. In this case, the content of inorganic salts such as chlorides and sulfates of metal cations in the europium compound (A) of the formula (1) is that as mentioned above. It is however preferable to use the europium compound in which the content of inorganic salts is controlled in a lower range.

The ink composition of the present invention is applied to or printed on paper, synthetic paper or a film by a known method such as usual bar coater coating, air knife coating, gravure coating, offset printing, flexographic printing, screen printing and ink jet printing.

Specific examples of recording material which can be used when the ink composition of the present invention is used in an ink jet recording method include postal matter including a post card and envelopes and information communication sheets such as paper and films. In addition to common paper, synthetic paper and films, those obtained by disposing an ink receptor layer on these base materials are also used. The ink receptor layer is formed by impregnating the above base material with a cationic polymer or by applying the cationic polymer to the base material, or by applying inorganic fine particles, such as porous silica, alumina sol and specific ceramics, which can absorb dyes in ink, to the surface of the above base material together with a hydrophilic polymer such as polyvinyl alcohol and polyvinylpyrrolidone. Also, fluorescent dyes are sometimes applied to improve whiteness. Also, the above base material may be color-printed or colored. It is usually known that an image is clearer and water resistance is stronger in the case of utilizing the ink composition for information communication sheets provided with an ink receptor layer. The dry film thickness after printing is usually about 0.001 to 10µm (0.001 to 10 g/m² in weight) and preferably about 0.005 to 3µm although this differs depending on printing system.

In order to record in a recording material by using the ink composition of the present invention and an ink jet recording method, a container stored with the ink composition of the present invention may be set to a known ink jet printer to carry out recording using a usual ink jet recording method. Examples of ink jet systems to which the ink composition of the present invention may be applied include a piezo system utilizing mechanical vibration and bubble jet (registered trade mark) system utilizing bubbles produced by heating. The film thickness obtained in an ink jet recording method is preferably about 0.005 to 0.2 µm.

The aqueous ink composition of the present invention is highly stable and free from a reduction in emission intensity with time even in a mixed solvent having high water content and is made into a coating film superior in friction resistance, water resistance and light fastness.

Accordingly, the ink composition of the present invention can be made into ink with reduced odor of the solvent and reduced flammability in consideration of an influence on environment. Also, the composition is free from the conventional problems concerning the drawbacks caused by inferior stability, specifically, clogging of a nozzle in an ink jet printer and sedimentation during coating, and therefore has high utility value as a low-pollution aqueous ink composition which may be utilized as print products relating to concealed characters and security. Also, because the aqueous ink composition of the present invention has good reducibility in water, it can be easily washed away with water when equipment for producing ink and a recording device using the ink are cleaned.

The europium compound of the present invention has high solubility in a mixed solvent having high water content and also has good preserving stability. Therefore, the aqueous ink composition using this compound has high stability even in a mixed solvent having high water content and is not reduced in emission intensity with time. Also, since the emission intensity is high, the content of the europium compound (complex) in the ink composition can be reduced. Further, the coating film of the ink composition has good friction resistance and water resistance. Also, the ink composition imparts good light fastness to print products.

Accordingly, the ink composition of the present invention can be made into ink with reduced odor of the solvent and reduced flammability in consideration of an influence on environment. Also, the composition is free from the conventional problems concerning the drawbacks caused by inferior stability, specifically, clogging of a nozzle in an ink jet printer and sedimentation during coating, and therefore has high utility value as a low-pollution aqueous ink composition which may be utilized as print products relating to concealed characters and security.

Also, because the europium compound (complex) of the present invention has high water solubility, it can be easily washed away with water when equipment for producing the europium compound and ink using the europium compound and a recording device using the ink are cleaned.

### Examples

The present invention will be explained in more detail by way of examples, but which do not intend to limit the present invention, in which all designations of parts indicate parts by weight.

### Example 1 (Synthesis of Compound No. (1))

70 parts of ethanol, 11 parts of 4,4,4-trifluoromethyl-1-(2-thienyl)-1,3-butanedione and 11.8 parts of an aqueous 10% lithium hydroxide solution were mixed at ambient temperature. To this solution was added dropwise an aqueous solution prepared by dissolving 4.5 parts of europium chloride hexahydrate in 10 parts of water and the mixture was stirred for 3 hours. After the reaction was finished, the reaction solution was added dropwise to 1000 g of ice water. The precipitated white solids were collected by filtration and dried to obtain 11 parts of a compound (2) represented by the following formula. Absorption spectrum (methanol): maximum absorption wavelength: 334 nm, Fluorescent spectrum (methanol): excitation maximum wavelength: 334 nm, fluorescent maximum wavelength: 616 nm.

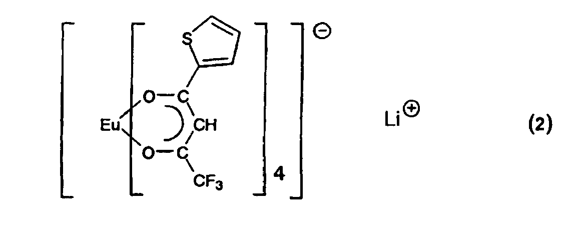

### Example 2 (Synthesis of Compound No. (2))

70 parts of ethanol, 11 parts of 4,4,4-trifluoromethyl-1-(2-thienyl)-1,3-butanedione and 19.8 parts of an aqueous 10% sodium hydroxide solution were mixed at ambient temperature. To this solution was added dropwise an aqueous solution prepared by dissolving 4.5 parts of europium chloride hexahydrate in 10 parts of water and the mixture was stirred for 3 hours. After the reaction was finished, the reaction solution was added dropwise to 1000 g of ice water. The precipitated white solids were collected by filtration and dried to obtain 11 parts of a compound (3) represented by the following formula.

Absorption spectrum (methanol): maximum absorption wavelength: 344 nm

Fluorescent spectrum (methanol): excitation maximum wavelength: 344 nm, fluorescent maximum wavelength: 616 nm.

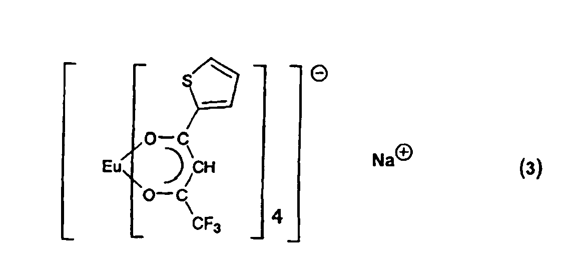

### Example 3 (Synthesis of Compound No. (1))

82 parts of ethanol, 11 parts of 4,4,4-trifluoromethyl-1-(2-thienyl)-1,3-butanedione and 11.8 parts of an aqueous 10% lithium hydroxide solution were mixed at ambient temperature. To this solution was added dropwise an aqueous solution prepared by dissolving 4.5 parts of europium chloride hexahydrate in 43 parts of water and the mixture was stirred for 3 hours to obtain a solution of a compound represented by the following formula (2) (the theoretical value of the concentration of the compound represented by the formula (2) in the solution is 8.5%).

Absorption spectrum (methanol): maximum absorption wavelength: 334 nm

Fluorescent spectrum (methanol): excitation maximum wavelength: 334 nm, fluorescent maximum wavelength: 616 nm.

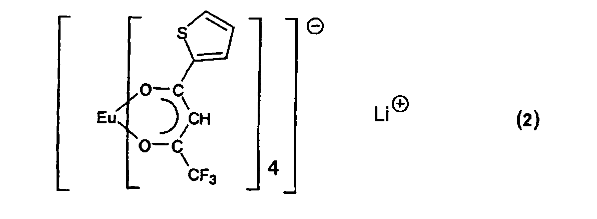

### Example 4 (Synthesis of Compound No. (2))

82 parts of ethanol, 11 parts of 4,4,4-trifluoromethyl-1-(2-thienyl)-1,3-butanedione and 19.8 parts of an aqueous 10% sodium hydroxide solution were mixed at ambient temperature. To this solution was added dropwise an aqueous solution prepared by dissolving 4.5 parts of europium chloride hexahydrate in 43 parts of water and the mixture was stirred for 3 hours to obtain a solution of a compound represented by the following formula (3) (the theoretical value of the concentration of the compound represented by the formula (3) in the solution is 8.2%).

Absorption spectrum (methanol): maximum absorption wavelength: 344 nm

Fluorescent spectrum (methanol): excitation maximum wavelength: 344 nm, fluorescent maximum wavelength: 616 nm.

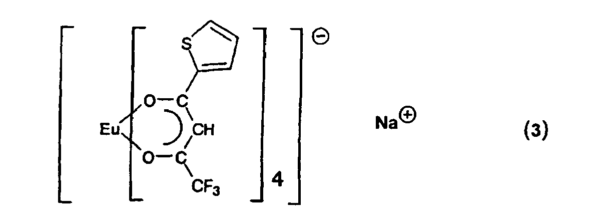

### Example 5 (Synthesis of Compound No. (11))

A compound No. (11) was obtained in the same manner as in Example 3 except that 11 parts of 4,4,4-trifluoromethyl-1-(phenyl)-1,3-butanedione was used in place of 11 parts of 4,4,4-trifluoromethyl-1-(2-thienyl)-1,3-butanedione (the theoretical value of the concentration of the compound No. (11) in the solution is 8.3%).

Fluorescent spectrum (methanol): excitation maximum wavelength: 341 nm, fluorescent maximum wavelength: 615 nm.

### Example 6 (Synthesis of Compound No. (14))

A compound No. (14) was obtained in the same manner as in Example 3 except that 10 parts of 4,4,4-trifluoromethyl-1-(2-furoyl)-1,3-butanedione was used in place of 11 parts of 4,4,4-trifluoromethyl-1-(2-thienyl)-1,3-butanedione (the theoretical value of the concentration of the compound No. (14) in the solution is 8.0%).

Fluorescent spectrum (methanol): excitation maximum wavelength: 355 nm, fluorescent maximum wavelength: 615 nm.

### Comparative Example 1 (Synthesis of a comparative compound)

In Example 4, 3 parts of 1-(3-sulfopropyl)pyridinium hydroxybetaine was added after the aqueous solution of europium chloride hexahydrate was added dropwise and the mixture was stirred for 3 hours to obtain a comparative compound represented by the following formula (4) (Compound No. 5 described in Patent Document 3) (the theoretical value of the concentration of the compound of the formula (4) in the solution is 9.2%).

Fluorescent spectrum (methanol): excitation maximum wavelength: 342 nm, fluorescent maximum wavelength: 616 nm.

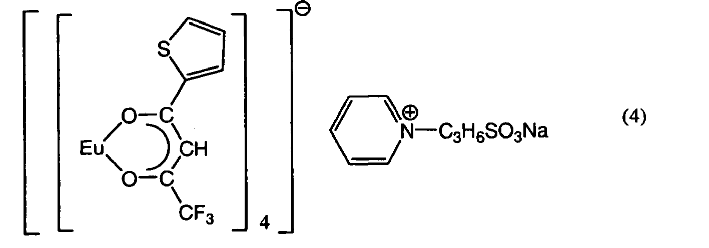

### Example 7

| | |
|---|---|
| Ethanol | 60 parts |
| Water | 40 parts |
| KL-506 (Note 1) | 3 parts |
| Reaction solution of the compound No. (1) obtained in Example 3 | 16 parts |

| | |
|---|---|
| (Note 1): KL-506 (manufactured by Kuraray Co., Ltd.) carboxyl group-modified PVA (degree of polymerization: 600, degree of saponification: 74 to 80) | |

The above components were mixed and dissolved and the obtained solution was subjected to precision filtration using a 0.45µ membrane filter to obtain an aqueous ink composition according to the present invention. This ink composition was applied to white PET Lumirror E22 manufactured by Toray by using a bar coater in a thickness of about 0.05 g/m² (film thickness: about 0.05µm) and dried to manufacture a colored body according to the present invention.

### Example 8

An aqueous ink composition and a colored body according to the present invention were obtained in the same manner as in Example 1 except that in Example 7, the reaction solution of the compound No. 2 obtained in Example 4 was used in place of the reaction solution of the compound No. 1 obtained in Example 3.

### Example 9

An aqueous ink composition and a colored body according to the present invention were obtained in the same manner as in Example 7 except that VA-37E (Note 2) was used in place of KL-506 in Example 7.
(Note 2): Lubiscole VA-37E (manufactured by BASF), ethanol solution containing 50% of a vinylpyrrolidone/vinyl acetate copolymer

### Example 10

| | |
|---|---|
| Ethanol | 40 parts |
| 1-Propanol | 35 parts |
| Water | 20 parts |
| K-30 (Note 8) | 3 parts |
| Reaction solution of the compound No. (11) obtained in Example 5 | 16 parts |

| | |
|---|---|
| (Note 8): Lubiscole K-30 (manufactured by BASF); polyvinylpyrrolidone | |

An aqueous ink composition and a colored body according to the present invention were obtained in the same manner as in Example 7 except that the above components were used.

### Example 11

An aqueous ink composition and a colored body according to the present invention were obtained in the same manner as in Example 10 except that in Example 10, the reaction solution of the compound No. 14 obtained in Example 6 was used in place of the reaction solution of the compound No. 11 obtained in Example 5.

### Comparative Example 2

A comparative aqueous ink composition and a comparative colored body were obtained in the same manner as in Example 10 except that in Example 10, the reaction solution of the compound synthesized in Comparative Example 1 was used in place of the reaction solution of the compound No. 11 obtained in Example 5.

The aqueous ink compositions and colored bodies obtained in this manner were subjected to various evaluation tests. Each test result is shown in Table 2. Also, as the evaluation standard, those described below were adopted.

**Table 2 Results of evaluation**

| | Condition of ink | | Emission intensity | |
|---|---|---|---|---|
| | Just after manufactured | After stored | Just after manufactured | After stored |
| Example 7 | ○ | ○ | 450 | 446 |
| Example 8 | ○ | ○ | 428 | 417 |
| Example 9 | ○ | ○ | 702 | 785 |
| Example 10 | ○ | ○ | 680 | 658 |
| Example 11 | ○ | ○ | 710 | 715 |
| Comparative | ○ | ○ | 435 | 428 |
| Example 2 | | | | |

| | Friction resistance | Water resistance | Light fastness | Adaptability to an ink jet |
|---|---|---|---|---|
| Example 7 | ○ | ○ | ○ | ○ |
| Example 8 | ○ | ○ | ○ | ○ |
| Example 9 | ○ | ○ | ○ | ○ |
| Example 10 | ○ | ○ | ○ | ○ |
| Example 11 | ○ | ○ | ○ | ○ |
| Comparative | ○ | ○ | ○ | ○ |
| Example 2 | | | | |

Explanations of each test method
(1) Condition of the ink (just after manufactured)
   The state of the resulting ink composition was observed visually to evaluate.
   ○: Stable in a uniformly dissolved or dispersed state.
   □: Though precipitates are observed, the redispersing ability is good and therefore, a uniform state is obtained by agitation.
   ×: Precipitation or separation is obtained.
(2) Condition of ink (after stored)
   The resulting ink composition was stored at 40°C for 2 hours to observe the state of the ink visually, thereby evaluating the stability.
   ○: Stable in a uniformly dissolved or dispersed state.
   □ : Though precipitates are observed, the redispersing ability is good and therefore, a uniform state is obtained by agitation.
   ×: Precipitation or separation is obtained.
(3) Emission intensity (just after the manufacturing of the ink)
   The emission intensity of the resulting colored body was measured. The measurement was made using a spectral fluorophotometer FP-6600 (manufactured by JASCO Corporation).
   In the measurement, the colored body was irradiated with 320 nm excitation light to measure the emission intensity of 615 nm light.
(4) Emission intensity (after stored)
   The obtained ink composition was stored at 40°C for 2 weeks and the emission intensity of the coating film was measured in the same manner as above.
(5) Friction resistance
   The obtained colored body was rubbed once in a forward and backward direction with a finger and then irradiated with ultraviolet rays by black light to confirm the emission of the colored body visually.
   ○: Good red emission was observed.
   ×: No emission was observed.
(6) Water resistance
   The obtained colored body including the base material was dipped in city water for one minute and dried. Then, the colored body was irradiated with ultraviolet rays by black light to confirm the emission of the colored body visually.
   ○: Good red emission was observed.
   ×: No emission or weak emission was observed.
(7) Light fastness
   The obtained colored body was applied to the inside wall of a room and was irradiated with ultraviolet rays after two weeks to observe the condition of emission.
   ○: Good red emission was observed.
   ×: No emission or weak emission was observed.
(8) Adaptability to an ink jet
   The ink composition obtained in each example was used in an ink jet printer (trade name: PICTY, manufactured by NEC Corporation, 100 L) to carry out bar code printing on common paper.
   ○: Proper bar code could be printed.
   ×: No proper bar code could be printed.

### Example 12

50 parts of water was added to 10 parts of the ink obtained in Example 10 according to the present invention to observe the state of the mixture, to find that the obtained mixture was a transparent solution. On the other hand, when 50 parts of water was added to 10 parts of the ink obtained in Comparative Example 2, the obtained mixture was clouded and precipitated with time. The ink of the present invention had good reducibility in water, whereas the comparative ink had unsatisfactory reducibility in water.

As is clear from Table 2 and Example 12, the aqueous ink composition of the present invention was highly stable, was not reduced in emission intensity with time and had satisfactory reducibility in water with time. Further, the colored body was superior in friction resistance, water resistance and light fastness.

## Claims

1. (Amended) An aqueous ink composition comprising a europium compound (A) represented by the following formula (1), a binder (B) and an aqueous medium (C): 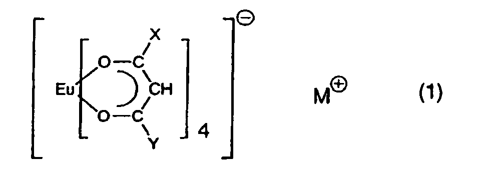 wherein X represents a benzene cyclic group, a naphthalene cyclic group, a pyridine cyclic group or a thiophene cyclic group which may have a substituent, Y represents a fluorinated hydrocarbon group having 1 to 10 carbon atoms and M represents an alkali metal or an alkali earth metal.

2. (Amended) The aqueous ink composition according to Claim 1, wherein X is a benzene cyclic group or a thiophene cyclic group.

3. The aqueous ink composition according to Claim 1 or 2, wherein Y is a trifluoromethyl group.

4. The aqueous ink composition according to any one of Claims 1 to 3, wherein M is an alkali metal.

5. The aqueous ink composition according to any one of Claims 1 to 4, wherein (B) is a water-soluble high-molecular compound.

6. The aqueous ink composition according to Claim 5, wherein (B) is polyvinyl alcohol, modified polyvinyl alcohol or a polymer having a cyclic amide group in a molecular structure.

7. The aqueous ink composition according to any one of Claims 1 to 6, wherein the content of (A) is 0.1 to 4% by weight, the content of (B) is 0.1 to 15% by weight and the content of (C) is 73.5 to 99.8% by weight.

8. A colored body treated with the aqueous ink composition as claimed in any one of Claims 1 to 7.

## Patentansprüche

1. Wässrige Tintenzusammensetzung umfassend eine Europium-Verbindung (A) dargestellt durch die folgende Formel (1), ein Bindemittel (B) und ein wässriges Medium (C): 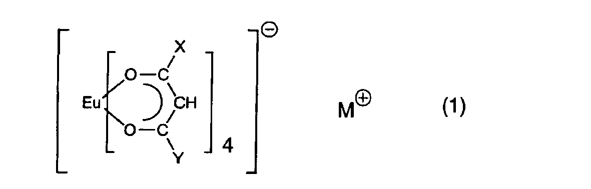 worin X eine zyklische Benzengruppe, eine zyklische Naphthalengruppe, eine zyklische Pyridingruppe oder eine zyklische Thiophengruppe darstellt, die einen Substituenten enthalten kann, Y eine fluorierte Kohlenwasserstoffgruppe mit 1 bis 10 Kohlenstoffatomen und M ein Alkalimetal oder ein Erdalkalimetall darstellt.

2. Wässrige Tintenzusammensetzung nach Anspruch 1, worin X eine zyklische Benzengruppe oder eine zyklische Thiophengruppe ist.

3. Wässrige Tintenzusammensetzung nach Anspruch 1 oder 2, worin Y eine Trifluormethylgruppe ist.

4. Wässrige Tintenzusammensetzung nach einem der Ansprüche 1 bis 3, worin M ein Alkalimetall ist.

5. Wässrige Tintenzusammensetzung nach einem der Ansprüche 1 bis 4, worin (B) eine wasserlösliche hochmolekulare Verbindung ist.

6. Wässrige Tintenzusammensetzung nach Anspruch 5, worin (B) Polyvinylalkohol, modifizierter Polyvinylalkohol oder ein Polymer ist, das eine zyklische Amidgruppe in der molekularen Struktur enthält.

7. Wässrige Tintenzusammensetzung nach einem der Ansprüche 1 bis 6, worin der Gehalt an (A) 0,1 bis 4 Gewichtsprozent, der Gehalt an (B) 0,1 bis 15 Gewichtsprozent und der Gehalt an (C) 73,5 bis 99,8 Gewichtsprozent beträgt.

8. Gefärbter Körper, der mit der wässrigen Tintenzusammensetzung nach einem der Ansprüche 1 bis 7 behandelt wurde.

## Revendications

1. Composition d'encre aqueuse comprenant un composé de l'europium (A) représenté par la formule (1) suivante, un liant (B) et un milieu aqueux (C) : 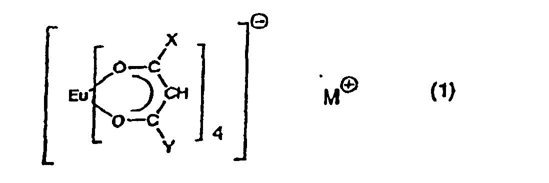 où X représente un groupe cyclique benzénique, un groupe cyclique naphtalène, un groupe cyclique pyridine ou un groupe cyclique thiophène qui peut avoir un substituant, Y représente un groupe hydrocarboné fluoré ayant 1 à 10 atomes de carbone et M représente un métal alcalin ou un métal alcalino-terreux.

2. Composition d'encre aqueuse selon la revendication 1 où X est un groupe cyclique benzénique ou un groupe cyclique thiophène.

3. Composition d'encre aqueuse selon la revendication 1 ou 2 où Y est un groupe trifluorométhyle.

4. Composition d'encre aqueuse selon l'une quelconque des revendications 1 à 3 où M est un métal alcalin.

5. Composition d'encre aqueuse selon l'une quelconque des revendications 1 à 4 où (B) est un composé de haute masse moléculaire soluble dans l'eau.

6. Composition d'encre aqueuse selon la revendication 5 où (B) est le poly(alcool vinylique), un poly(alcool vinylique) modifié ou un polymère ayant un groupe amide cyclique dans une structure moléculaire.

7. Composition d'encre aqueuse selon l'une quelconque des revendications 1 à 6 où la teneur de (A) est 0,1 à 4 % en masse, la teneur de (B) est 0,1 à 15 % en masse et la teneur de (C) est 73,5 à 99,8 % en masse.

8. Corps coloré traité avec la composition d'encre aqueuse selon l'une quelconque des revendications 1 à 7.
